# EUROPEAN PATENT APPLICATION

(11) **EP 2 537 920 A1**
(43) Date of publication of application: **26.12.2012**
(21) Application number: 11744619.5
(22) Date of filing: 15.02.2011
(51) Int. Cl.: C12N 5/00, A61L 27/00, C12N 5/0789

(54) **METHOD FOR PRODUCTION OF ARTIFICIAL PLURIPOTENT STEM CELL**

(30) Priority: 16.02.2010 JP 2010030830
(71) Applicant: Saitama Medical University, Iruma-gun, Saitama 350-0495 (JP)
(72) Inventor: HISHIDA, Tomoaki, Iruma-gun Saitama 350-0495 (JP); OKUDA, Akihiko, Iruma-gun Saitama 350-0495 (JP); KATO, Hidemasa, Iruma-gun Saitama 350-0495 (JP)
(74) Representative: Stephen, Robert John
(86) International application number: PCT/JP2011/053110
(87) International publication number: WO 2011/102333

(57) **Abstract**

Disclosed is a method for producing an iPS cell having a very similar gene expression pattern to that of an ES cell with high efficiency. Specifically disclosed is a method for producing an artificial pluripotent stem cell (an iPS cell), which comprises the steps of: introducing a pluripotency-inducing factor comprising at least an Myc family gene or an Myc family protein into a somatic cell; and culturing the somatic cell in the presence of a sirtuin inhibitor and/or a poly-ADP ribose polymerase (PARP) inhibitor.

## Description

### Technical Field

The present invention relates to a method for producing a true artificial pluripotent stem cell.

### Background Art

In recent years, the research of a cell having pluripotency, i.e. the ability to differentiate into any and all cell that constitutes an individual has been promoted with the objective to serve in regenerative medicine etc. One example of a cell having pluripotency is an embryonic stem cell (ES cell) obtained from an early embryo. An ES cell can be almost indefinitely proliferated while maintaining pluripotency to differentiate into theoretically any tissue.

However, when an ES cell is utilized for regenerative medicine, rejection reaction may be evoked because a cell generated from another person's fertilized egg is transplanted into the patient. In addition, there are ethical problems since a human embryo is destroyed for establishment. Accordingly, a reprogramming technology was desired in which a somatic cell that has once differentiated into a particular cell is dedifferented and allowed to acquire pluripotency comparable to an ES cell.

In recent years, technologies have been developed to generate an artificial pluripotent stem cell (induced pluripotent stem cell; iPS cell) having almost equal self-propagation and differentiation pluripotency as an ES cell by introducing a pluripotency inducer such as Oct3/4, Sox2, Klf4, and c-Myc into a somatic cell and forcing expression (see, e.g. Non-Patent Documents 1-3). According to methods described in Non-Patent Documents 1 and 2, four factors of Oct3/4, Sox2, c-Myc, and Klf4 are introduced as the pluripotency inducer to obtain iPS cells from mouse embryonic fibroblasts and human mature skin cells. On the other hand, in the method in Non-Patent Document 3, four factors of Oct4, Sox2, Nanog, and Lin28 are introduced as the pluripotency inducer to obtain iPS cells from human skin cells.

Since iPS cells can be generated from a patient's own somatic cells, if these are cultured to produce necessary cells, tissues, or organs, they can be transplanted to the patient without the problem of immune rejection. In addition, it is also expected to solve the ethical problems that were the obstacles for practical application of ES cells.

As a means to judge whether an iPS cell was reprogrammed, i.e. pluripotency was restored, there is a method of confirming whether or not a gene specifically expressed in an ES cell is expressed.
When Silva et al. introduced four pluripotency inducers of Oct3/4, Sox2, c-Myc, and Klf4 into a relatively reprogrammable neural stem cell by a retrovirus, appearance similar to ES cells was presented after three days, and after two more days the plate was confluent with ES cell-like cells and passaging was necessary. However, when the expression of four ES cell markers of Fgf4, Rex1, Nanog, and endogenous Oct3/4 were verified at a transcriptional level in these cells, it was found that all were expressed but at a lower expression level compared to ES cells. In addition, Nanog protein was confirmed to be expressed only in some cells by immunostaining (see Non-Patent Document 4).

As described above, since iPS cells generated with a conventional method comprise many cell colonies judged to have insufficient reprogramming (hereinafter referred to as "partial iPS cells"), screening from a large number of cell colonies is necessary in order to obtain true iPS cells. However, in the current technology standard, since the establishment efficiencies of not only true iPS cells but also partial iPS cells are extremely low, it is also difficult to obtain and screen numerous colonies.

It was recently reported from multiple laboratories that the induction efficiency of iPS cells is increased by introducing the shRNA against antioncogene p53 into a cell into which four pluripotency inducers of Oct3/4, Sox2, Klf4, and c-Myc were introduced (see Non-Patent Documents 5 and 6). However, since the iPS cell obtained with this method has the p53 gene that acts most suppressively on tumor production knocked down the possibility of high risk of tumorigenesis after transplantation is high, and practical application is thought to be difficult.

Various agents such as valproic acid are also reported to increase the efficiency of iPS cell induction (see Non-Patent Documents 7 and 8). However, these agents are anticipated to act on an extremely broad group of molecules within cells, and detailed investigation regarding for example side effects of employing these agents is thought to be necessary in order to employ them in transplantation.

On the other hand, in the conventional method, the above pluripotency inducers are introduced into a cell with a viral vector. In the method, as a result of the protooncogene c-Myc gene being incorporated into the host DNA, it is likely to be reactivated in the cell. Therefore, iPS cell induction by a method of introducing the c-Myc gene with a plasmid vector or a non-inserted recombinant viral vector or a method of directly introducing a protein into the cell is also proposed. According to these methods, the c-Myc gene is not incorporated into the host genome and carcinogenicity can be suppressed. In these methods, however, there was a problem that the efficiency of iPS cell induction is significantly reduced (see Non-Patent Documents 9-11).

### Citation List

Non-Patent Document 1
   Takahashi K, Yamanaka S. (2006) Cell 126: 663-676.
Non-Patent Document 2
   Takahashi K, Tanabe K, Ohnuki M, Narita M, Ichisaka T, Tomoda K, Yamanaka S. (2007) Ce11131: 861-872.
Non-Patent Document 3
   Yu J, et al. (2007) Science 318: 1917-1920.
Non-Patent Document 4
   Silva J. et al. (2008) PLoS Biology 6 (10): e253
Non-Patent Document 5
   Kawamura T, et al. (2009) nature 460: 1140-1144.
Non-Patent Document 6
   Hong H, et al. (2009) nature 460: 1132-1135.
Non-Patent Document 7
   Huangfu D, et al. (2008) Nat. Biotechnol.26:795-797.
Non-Patent Document 8
   Shi Y, et al. (2008) Cell Stem Cell 3: 568-574.
Non-Patent Document 9
   Okita K, et al. (2008) Science.322:949-953.
Non-Patent Document 10
   Stadtfeld M, et al. (2008) Science 322: 945-949.
Non-Patent Document 11
   Zhou H, et al. (2009) Cell Stem Cell 4: 381-384.

### Summary of the Invention

### Problems to be Solved by the Invention

The object of the present invention is to provide a method for efficiently producing an iPS cell having a very similar gene expression pattern to that of an ES cell.

### Means for Solving the Problems

As a result of repeated research to solve the above problems, the present inventors found that: the induction efficiency is significantly increased when a somatic cell into which a pluripotency inducer is introduced is cultured in the presence of nicotinamide; the effect is obtained when the Myc family is included as the pluripotency inducer; nicotinamide increases the induction efficiency of iPS cells by suppressing the function of sirtuin or poly-ADP ribose polymerase; and nicotinamide treatment is desirably performed within a few days after introducing the pluripotency inducer into a somatic cell, i.e. at an early stage of induction.
Further, it was found that combining these methods with the 2i method conventionally known to transform a partial iPS cell into a true iPS cell (Silva, J. et al. (2008) PLoS Biology 6 (10): e253) will dramatically increase the induction efficiency of true iPS cells.
It was also confirmed that when performing the 2i method, transformation efficiency into true iPS cells is increased by extending the time from introduction of the pluripotency inducer into a somatic cell until initiation of treatment by MEK inhibitor and GSK-3β inhibitor to longer than a given number of days.
In other words, the present invention relates to:
[1] a method for producing an artificial pluripotent stem cell (iPS cell) comprising the steps of: introducing a pluripotency inducer comprising at least an Myc family gene or an Myc family protein into a somatic cell; and culturing said somatic cell in the presence of a sirtuin inhibitor and/or a poly-ADP ribose polymerase (PARP) inhibitor;
[2] the method according to the above [1], wherein the step of culturing said somatic cell in the presence of said sirtuin inhibitor and/or PARP inhibitor is, counting the day of performing the step of introducing said pluripotency inducer into the somatic cell as Day 0, terminated before Day 10;
[3] the method according to the above [1] or [2], wherein the step of culturing said somatic cell in the presence of said sirtuin inhibitor and/or PARP inhibitor is, counting the day of performing the step of introducing said pluripotency inducer into the somatic cell as Day 0, initiated on or after Day 1;
[4] the method according to any one of the above [1] to [3], wherein said sirtuin inhibitor and/or PARP inhibitor is nicotinamide;
[5] the method according to the above [4], wherein the step of culturing said somatic cell in the presence of said sirtuin inhibitor and/or PARP inhibitor comprises adding said nicotinamide at a concentration of 1 mM to 10 mM to a medium for said somatic cell;
[6] the method according to any one of the above [1] to [5], which comprises, after the step of culturing said somatic cell in the presence of said sirtuin inhibitor and/or PARP inhibitor, a step of further culturing said somatic cell in the presence of a GSK-3 inhibitor and an MEK inhibitor;
[7] the method according to the above [6], wherein the step of further culturing said somatic cell in the presence of said GSK-3 inhibitor and MEK inhibitor is, counting the day of performing the step of introducing said pluripotency inducer into the somatic cell as Day 0, initiated on or after Day 5;
[8] the method according to the above [1] to [7], wherein said Myc family gene or Myc family protein is a c-Myc gene or L-Myc gene, or a c-Myc protein or L-Myc protein;
[9] the method according to any one of the above [1] to [8], wherein said pluripotency inducer further comprises one or more genes selected from the group consisting of Oct3/4 gene, Klf4 gene, Sox2 gene, Nanog gene and LIN28 gene;
[10] a method for producing an artificial pluripotent stem cell (iPS cell) comprising the steps of: introducing a pluripotency inducer into a somatic cell; and further culturing said somatic cell in the presence of a GSK-3 inhibitor and an MEK inhibitor; wherein the step of further culturing said somatic cell in the presence of said GSK-3 inhibitor and MEK inhibitor is, counting the day of performing the step of introducing said pluripotency inducer into the somatic cell as Day 0, initiated on or after Day 5;
[11] the method according to the above [10], wherein said pluripotency inducer is one or more genes selected from the group consisting of Oct3/4 gene, Klf4 gene, Sox2 gene, c-Myc gene, L-Myc gene, Nanog gene, and Lin28 gene;
[12] The method according to [1] to [11], wherein said artificial pluripotent stem cell (iPS cell) is a mouse or human-derived cell;
[13] an artificial pluripotent stem cell (iPS cell) produced by the method according to any one of the above [1] to [12]; and
[14] A culture kit for an artificial pluripotent stem cell (iPS cell), comprising a sirtuin inhibitor or poly-ADP ribose polymerase (PARP) inhibitor, a GSK-3 inhibitor, and an MEK inhibitor.

### Advantages of the Invention

According to the method for producing an iPS cell of the present invention, a true iPS cell having a very similar gene expression pattern to that of an ES cell can be efficiently obtained.
If nicotinamide, a type of vitamin, is employed as the sirtuin inhibitor and/or PARP1 inhibitor, side effects on cells are extremely low, and iPS cell which is safe for transplantation can be obtained. Nicotinamide is also inexpensive and therefore easily put to practical application.
Further, according to a method that combines nicotinamide and the 2i method, the production rate of the true iPS cell can be further increased, and the need for screening can be reduced. The induction speed is also significantly improved, and thus it is thought that the use of a feeder cell derived from other species employed for increasing the induction efficiency can be withheld, and the safety of the iPS cell can be further increased.

### Brief Description of the Drawings

Figure 1 is the result of inducing mouse iPS cells in the presence of nicotinamide. In cells into which four factors of Oct3/4, Sox2, Klf4, and c-Myc were introduced (4F (c-Myc)), the induction efficiency of iPS cells was significantly increased (Figures 1A and B), but in cells into which three factors excluding c-Myc were introduced (3F), no improvement in induction efficiency was seen (Figure 1C);
Figure 2 is the result of GFP detection in mouse iPS cells induced in the presence or absence of nicotinamide. The green color is weak before selection with puromycin (upper column), showing that a large amount of partial iPS cells are contained;
Figure 3 is the result of varying the administration timing of nicotinamide and confirming the influence on mouse iPS cell induction. The induction efficiency was confirmed to be significantly increased by culturing in the presence of nicotinamide at an early stage of induction;
Figure 4 is a photograph showing the appearance on Day 4 of induction when cultured in the presence or absence of nicotinamide at an early stage of induction. Mouse iPS cell-like colonies were already observed on Day 4 when cultured in the presence of nicotinamide;
Figure 5 is the result of counting the number of cells and the number of AP-positive colonies for each cases of Figure 4. In the nicotinamide treatment group, an increase in the number of cells and a significant increase in the number of AP-positive colonies were acknowledged;
Figure 6 shows the result of inducing mouse iPS cells in the presence of resveratrol which activates sirtuin. The induction efficiency was significantly reduced when cultured in the presence of resveratrol at an early stage of induction;
Figure 7 shows the result of inducing mouse iPS cells with the 2i method after nicotinamide treatment or after no treatment. The induction efficiency of true iPS cells was confirmed to be significantly increased by combining nicotinamide treatment and the 2i method (Figure 7D);
Figure 8 is the result of confirming the influence on mouse iPS cell induction by varying the timing of the 2i method initiation. It was found that the induction efficiency is the highest when initiation was on Day 13 after induction;
Figure 9 is the result of confirming the influence on iPS cell induction by performing the 2i method after nicotinamide treatment. It was found that an induction efficiency of about 10 times that compared to the conventional method is obtained when the two methods are combined. It was also confirmed that the induction efficiency is increased by three times or more when nicotinamide was added from Day 1 after induction compared to when the addition was from Day 0 (Figures 9A and 9B); and
Figure 10 shows the result of inducing human iPS cells in the presence or absence of nicotinamide. The induction efficiency
of iPS cells increased when either of c-Myc and L-Myc was employed as the Myc family gene.

### Description of Embodiments

The embodiments of the present invention will now be described.

### (First Aspect of Method for Producing an iPS Cell)

The first aspect of the method for producing an iPS cell of the present invention is characterized in that it comprises the steps of: introducing a pluripotency inducer comprising at least an Myc family gene or an Myc family protein into a somatic cell; and culturing the somatic cell in the presence of a sirtuin inhibitor and/or a poly-ADP ribose polymerase (PARP) inhibitor.

### (Step of Introducing a Pluripotency Inducer)

The term "iPS cell" is used herein in its broadest sense, and means a cell which has acquired the differentiation potency to a desired cell and the desired self-propagation by introducing a pluripotency inducer into a somatic cell. The differentiation pluripotency and the self-propagation of an iPS cell is preferably equal to that of an ES cell, but even if it is lower, a cell is included in an iPS cell in the present invention as long as it possesses the differentiation potency to a desired cell and the desired self-propagation.
Any cell obtained by introducing a pluripotency inducer into a somatic cell may be referred to herein as an iPS cell. In this case, an iPS cell includes a partial iPS cell with insufficient reprogramming. In order to discriminate a partial iPS cell and an iPS cell in a state with sufficient reprogramming, the latter may be referred to as a "true iPS cell," but these are relative terms and do not mean any particular absolute state.
An iPS cell in a state with sufficient reprogramming to achieve a given objective is considered a true iPS cell. For example, when the objective is differentiation into a cell constituting a given organ, a cell is considered a true iPS cell in the present invention even if it does not possess the differentiation potency into any and all other cell as long as it is an iPS cell that possesses the differentiation potency into the cell.

Whether a cell is an iPS cell or not can be determined by those skilled in the art according to well-known methods. For example, the cell can be determined to be an iPS cell by confirming that it is morphologically equal to an ES cell, or it can be determined to be an iPS cell by applying a well-known differentiation induction method and culturing the cell in vitro to confirm that it can differentiate into a desired cell.
When the cell is a mouse cell, it may be determined to be an iPS cell by injecting the cell into a fertilized egg and confirming that a chimeric mouse can be generated.
The cell can also be determined to be an iPS cell by subcutaneously transplanting it into an immunodeficient mouse, and analyzing the tumor tissue formed after a given amount of time to confirm that it is a teratoma with a mixture of various tissues such as nerve, skin, and muscle.
The cell may be determined to be an iPS cell by confirming the expression therein of an undifferentiated marker such as a marker gene specifically expressed in ES cells. Examples of a marker gene specifically expressed in ES cells include Fbx15, Nanog, Fgf4, Rex1, and Oct3/4. Alkaline phosphatase is also an undifferentiated marker, and positive alkaline phosphatase staining can be confirmed to determine that the cell is in an undifferentiated state.
A genome-wide gene expression pattern is detected with for example a microarray, and those having high correlation with the expression pattern of an ES cell may also be determined to be an iPS cell.
The expression property of a cell surface antigen is compared to an ES cell, and those having high correlation can also be determined to be an iPS cell.
The DNA methylation in the cell may further be detected and compared with an ES cell to confirm the similarity with an ES cell.
The decision of whether or not a cell is an iPS cell can be made by at least one of the above methods, but two or more can be combined for making the decision. For example, it is preferred that the decision of a cell being an iPS cell is based on the following four factors: the morphology is similar to an ES cell; the expression of an undifferentiated marker is observed; the possession of in vitro differentiation ability; and the possession of teratoma formation ability. In addition to these four factors, it is more preferred to confirm whether a direct injection into a blastocyst can generate a chimeric mouse.

A specific example of selecting an iPS cell that is closer to an ES cell includes a method of using the Nanog gene expression as indication (Okita et al., (2007) Nature 448, doi: 10. 1038/nature05934). In this method, a Bacterial Artificial Chromosome (BAC) having a Nanog gene placed in the center is isolated, and a Green Fluorescent Protein (GFP) gene, an internal ribosome entry site (IRES), and a puromycin resistance gene (Puro^{r}) are inserted into the 5' untranslated region of the Nanog gene.
GFP is expressed when such a construct is introduced into an ES cell, but is not expressed in a differentiated cell. Similarly, when a pluripotency inducer is introduced into a mouse fetal fibroblast, and the construct is simultaneously introduced, a cell expressing the Nanog gene, i.e. a sufficiently reprogrammed iPS cell presents GFP-positive and acquires puromycin resistance. Therefore, the iPS cell can be selected by adding puromycin to the medium. According to this method, an iPS cell showing proliferation ability, gene expression, and DNA methylation equal to that of an ES cell can be obtained.

A pluripotency inducer herein is a factor that can restore pluripotency to the somatic cell by being introduced into a somatic cell. Examples of a pluripotency inducer include Oct3/4, Klf family genes (such as Klf1, Klf2, Klf4, and Klf5 etc.), Myc family genes (such as c-Myc, N-Myc, and L-Myc etc.), Sox family genes (such as Sox1, Sox2, and Sox3 etc.), Nanog gene, and Lin28 gene.
As described below, although it is essential to employ an Myc family gene or an Myc family protein as the pluripotency inducer in the first aspect of the method for producing an iPS cell according to the present invention, as other pluripotency inducers, it is preferred to introduce a combination comprising at least two out of three genes of Oct3/4, Klf4, and Sox2.
The introduction of a combination of four genes of Oct3/4, Sox2, Nanog, and Lin28 other than the Myc family is also preferred.

In addition, one or more genes selected from the group consisting of Fbx15, E-Ras, ECAT15-2, Tcl1, β-catenin, ECAT1, Esg1, Dnmt3L, ECAT8, Gdf3, Sox15, ECAT15-1, Fthl17, Sal14, Rex1, UTF1, Stella, Stat3, and Grb2 may also be introduced as the pluripotency inducer.
The pluripotency inducer is not limited to genes, but may be a protein expressed by the above genes (e.g. an Myc family protein expressed by an Myc family gene (such as c-Myc, N-Myc, L-Myc etc.)) or other compounds, and a combination of genes, proteins, and/or compounds may also be employed.

In the present invention, the method for introducing the pluripotency inducer into a somatic cell is not particularly limited, but when the pluripotency inducer is a gene, a vector suitable for the host somatic cell can be used. Examples include, but are not limited to, a viral vector such as a retrovirus or lentivirus, a plasmid vector, a non-inserted recombinant viral vector, and an artificial chromosome vector (such as Yeast artificial chromosome; YAC, bacterial artificial chromosome; BAC, or P1-derived artificial chromosome; PAC etc.).
With a viral vector, a gene can be efficiently introduced by infecting a cell with a virus. However, the exogenous gene may be reactivated as a result of random integration into the host DNA. Consequently, when a protooncogene etc. is introduced as the pluripotency inducer, the method of introduction with a plasmid vector, a transposon, or a non-inserted recombinant viral vector, or the method of introducing a protein is preferred. These vectors and proteins can be introduced into a cell by a well-known method.

A somatic cell herein is a generic term for any cell constituting an organism excluding the germ cell. The type of somatic cell employed in the method of the present invention is not particularly limited, and may be a somatic cell in the fetal stage or an adult somatic cell. It may be a differentiated cell such as an epithelial cell, a fibroblast, a chondrocyte, a myocyte, a cardiomyocyte, and a hepatocyte, or an adult stem cell such as a hematopoietic stem cell, a neural stem cell, a mesenchymal stem cell, a hepatic stem cell, a pancreatic stem cell, a skin stem cell, a muscle stem cell, and a genital tract cell. A cell derived from a tissue such as umbilical cord blood or umbilical cord as well as placenta may also be employed. After collecting these cells from an organism, they can be established with a well-known method in an appropriate medium.
A somatic cell derived from any and all mammals can be employed, although that derived from e.g. human, mouse, rat, rabbit, hamster, cow, horse, and sheep etc. are preferred.

When an iPS cell obtained with the method of the present invention is employed for disease therapy, it is preferred to employ a somatic cell collected from the patient, for example, a fibroblast established from easily collected skin can be employed.
A disease-specific iPS cell can also be established with a somatic cell collected from a patient having a particular disease. A disease-specific iPS cell is useful for researching the onset mechanism or for developing a therapeutic drug.

In the method for producing an iPS cell according to the present invention, at least an Myc family gene or an Myc family protein is introduced into a somatic cell as the pluripotency inducer. Among the Myc family genes, c-Myc gene and N-Myc gene are transcriptional regulators involved in cell differentiation and proliferation, and are reported to be involved in maintaining pluripotency.
When the c-Myc gene is introduced into a cell as the pluripotency inducer, it may be integrated into the host DNA and reactivated to form a tumor, but it is known that the induction efficiency of iPS cells will be significantly reduced if the c-Myc gene is not employed (e.g. Silva, J. et al, PLoS Biology 2008 Oct 21;6 (10):e253).

Reactivation of the c-Myc gene can be prevented by employing a method where the c-Myc gene will not be integrated into the genome DNA of the somatic cell when being introduced into the somatic cell. Examples of such methods include the methods of employing a plasmid vector, a transposon, or a non-inserted recombinant viral vector etc. as the vector, but the induction efficiency of iPS cells will be reduced by using of these vectors. The c-Myc protein instead of the c-Myc gene can also be introduced to induce an iPS cell, but the induction efficiency of this method is also low.
According to the method of the present invention, however, it is possible to significantly increase the reduced induction efficiency, and thus safe and sufficiently reprogrammed iPS cell can be efficiency obtained.
Alternatively, N-Myc or L-Myc gene as the Myc family gene can also be introduced into a somatic cell as the pluripotency inducer. The L-Myc gene in particular is reported to be less carcinogenic compared to the c-Myc gene, and a safe iPS cell with suppressed carcinogenicity relative to an iPS cells by introduction of the c-Myc gene can be induced. Further, because the L-Myc gene has low carcinogenicity, it can be integrated into the host DNA with a method having high introduction efficiency which is preferable. As described above, by employing a method for introducing a pluripotency inducer with high induction efficiency to carry out the iPS cell induction method of the present invention, a higher induction efficiency of iPS cells can be obtained.

### (Step of Culturing in the Presence of Sirtuin Inhibitor and/or PARP Inhibitor)

The method for producing an iPS cell according to the present invention comprises, after the step of introducing a pluripotency inducer, a step of culturing the somatic cell in the presence of a sirtuin inhibitor and/or a PARP inhibitor.
Sirtuin is a group of NAD-dependent deacetylases, an example of which includes SIRT1. SIRT1 is an enzyme involved in DNA repair or transcriptional regulation, and uses histone, p53, and NFκB etc. as the substrate. PARP is an enzyme that catalyzes the poly-ADP ribosylation reaction which has an important role in DNA repair or transcriptional regulation.
The sirtuin inhibitor and/or PARP inhibitor is preferably added at a relatively early stage after introduction of the pluripotency inducer. For example, counting the day of introduction as Day 0, it is added daily to the medium for less than ten days after introduction, preferably less than eight days, further preferably less than four days. It is also preferred for example to add the sirtuin inhibitor and/or PARP inhibitor daily after introduction of the pluripotency inducer until when it first becomes confluent and trypsin-treated.
On the other hand, the sirtuin inhibitor and/or PARP inhibitor is, counting the day of introduction of the pluripotency inducer as Day 0, preferably initiated on or after Day 1. By initiating on or after Day 1, the induction efficiency of iPS cells can be significantly increased.

In the method according to the present invention, examples of the sirtuin inhibitor and/or PARP inhibitor include Sirtinol, Cambinol, AGK2, and Splitomicin which are sirtuin inhibitors, 3-Aminobenzamide, DPQ, and NU1025 which are PARP inhibitors, as well as nicotinamide which is a sirtuin and PARP inhibitor, and in particular nicotinamide is preferably employed.
Nicotinamide is a type of vitamin B3, and is a precursor of nicotinamide adenine dinucleotide (NAD) which is a coenzyme for redox reaction. Because it is a substance intrinsically utilized in vivo, side effects on cells are thought to be extremely low, and an iPS cell which is safe for transplantation can be obtained. It is also an inexpensive agent, making it suitable for mass production as well.

The sirtuin inhibitor and/or PARP inhibitor may be added to the medium of the cell which has the pluripotency inducer introduced. The concentration of the sirtuin inhibitor and/or PARP inhibitor added is not particularly limited, for example, about 1 mM to about 10 mM, preferably about 3 mM to about 5 mM, and most preferably about 4 mM for nicotinamide, and the concentrations for other sirtuin inhibitors and/or PARP inhibitors can also be appropriately selected by those skilled in the art.

Other culture conditions in this step (such as composition and temperature of the medium) can be appropriately selected by those skilled in the art.

### (Step of Culturing in the Presence of GSK-3 Inhibitor and MEK Inhibitor)

In the method for producing an iPS cell according to the present invention, it is preferred, after the step of culturing in the presence of a sirtuin inhibitor and/or a PARP inhibitor, to further culture the cell in the presence of a GSK-3 inhibitor and an MEK inhibitor.

A GSK-3 inhibitor refers to an inhibitor that targets one or more members of the glycogen synthase kinase 3 (GSK-3) family. Well-known GSK3 family members are, not limited to, GSK-3α and GSK-3β. The inhibitor of GSK-3β is a particularly preferred as GSK-3 inhibitor employed in the present invention.
Examples of GSK-3 inhibitors include CHIR98014, CHIR99021, AR-AO144-18, TDZD-8, SB216763, and SB415286. CHIR99021 and CHIR98014 are particularly preferred in terms of specificity towards GSK-3. The concentration of the GSK-3 inhibitor added is not particularly limited, for example, CHIR99021 is preferably added to the medium at a concentration of 0.01 µM to 100 µM, more preferably 0.1 µM to 20 µM, and further preferably 0.3 µM to 10 µM. The concentrations for other GSK-3 inhibitors can also be appropriately selected by those skilled in the art.
Nucleic acids such as siRNA, antisense, and ribozyme etc. may be used as the GSK-3 inhibitor. For example, if an siRNA comprising an RNA strand complementary to a part of the GSK-3 gene is introduced into a cell, GSK-3 mRNA can be degraded and expression of the GSK-3 protein can be inhibited.

An MEK inhibitor refers to an inhibitor that targets one or more members of the MAP kinase kinase (mitogen activated protein kinase/ERK kinase; MEK) family. Examples of MEK family members include MEK1, MEK2, and MEK3. Examples of MEK inhibitors include, but are not limited to, PD184352 and PD98059 which inhibit MEK1, as well as PD0325901, U0126, and SL327 etc. which inhibit MEK1 and MEK2. Among these, PD184352 and PD0325901 are particularly preferred since they have high specificity towards MEK and have high effect as inhibitors.
Nucleic acids such as siRNA, antisense, and ribozyme etc. may be used as the MEK inhibitor. For example, if an siRNA comprising an RNA strand complementary to a part of the MEK gene is introduced into a cell, MEK mRNA can be degraded and expression of the MEK protein can be inhibited.
The MEK inhibitor is preferably added to the medium at a concentration of 0.1 µM to 25 µM, more preferably 0.1 µM to 5 µM, and further preferably 0.2 µM to 2 µM.

In the step of culturing the cell in the presence of a GSK-3 inhibitor and an MEK inhibitor, it is also preferred to further add an antagonist of fibroblast growth factor receptor (FGFR) (such as SU5402 and PD173074 etc.) to the medium at a concentration of 0.1 µM to 20 µM, preferably 0.5 µM to 10 µM, and further preferably 1-5 µM.

The step of culturing the cell in the presence of a GSK-3 inhibitor and an MEK inhibitor is performed after the step of culturing the somatic cell in the presence of a sirtuin inhibitor and/or a PARP inhibitor as described above. The step of culturing the cell in the presence of a GSK-3 inhibitor and an MEK inhibitor is preferably on or after Day 5, more preferably on or after Day 7, further preferably on or after Day 9, and most preferably on or after Day 11 after the step of introducing a pluripotency inducer into the somatic cell.
On the other hand, the addition of the GSK-3 inhibitor and MEK inhibitor is preferably initiated before Day 15.

Other culture conditions in this step (such as composition and temperature of the medium) can be appropriately selected by those skilled in the art.

### (Second Aspect of Method for Producing an iPS Cell)

The second aspect of the method for producing an iPS cell of the present invention is characterized in that it comprises the steps of: introducing a pluripotency inducer into a somatic cell; and further culturing the somatic cell in the presence of a GSK-3 inhibitor and an MEK inhibitor; wherein the step of further culturing the somatic cell in the presence of a GSK-3 inhibitor and an MEK inhibitor is initiated on or after Day 5 after the step of introducing a pluripotency inducer into a somatic cell.

The step of culturing the cell in the presence of a GSK-3 inhibitor and an MEK inhibitor is initiated after the step of introducing a pluripotency inducer into the somatic cell, preferably on or after Day 5, more preferably on or after Day 7, further preferably on or after Day 10, most preferably on or after Day 12 and before Day 15.
Since culturing in the presence of a GSK-3 inhibitor and an MEK inhibitor is a relatively harsh condition for an ordinary cell, addition of GSK-3 inhibitor and MEK inhibitor at an early stage of induction is thought to evoke cell death.
Other terms employed in this aspect are synonymous to those in the first aspect as described above, and description is therefore omitted here.

Other culture conditions in this step (such as composition and temperature of the medium) can be appropriately selected by those skilled in the art.

### (Culture Kit)

The iPS cell culture kit of the present invention comprises at least a sirtuin inhibitor or a poly-ADP ribose polymerase (PARP) inhibitor, a GSK-3 inhibitor, and an MEK inhibitor. By employing a kit containing these at appropriate concentrations in separate containers, each reagent can be added in order to the medium of a somatic cell into which a pluripotency inducer is introduced, resulting in easy culture of iPS cells.
The kit preferably comprises other components of the medium, a culture vessel, and instruction for use etc.
The terms employed herein are for the purpose of describing particular embodiments and do not intend to limit the invention.

In addition, the term "comprising", "including", and "containing" as used herein, unless the content clearly indicates to be understood otherwise, intends that the stated item (such as parts, steps, elements, and numbers) exist, and do not exclude the existence of other items (such as parts, steps, elements, and numbers).
Unless otherwise defined, all terms used herein (including technical terms and scientific terms) have the same meaning as that broadly recognized by those skilled in the art of the technology the present invention belongs. The terms used herein, unless explicitly defined otherwise, should be construed to have meanings consistent to the meanings herein and in related technical fields, and are not to be construed as idealized or excessively formal meanings.
The embodiments of the present invention may be described referring to schematic diagrams. In such a case, they may be exaggerated in presentation in order to clarify the description.

The present invention will now be described in further detail referring to Examples. However, the present invention can be embodied by various aspects, and shall not be construed as being limited to the Examples described herein.

### Examples

### Test 1. Induction of Mouse iPS Cells in the Presence of Nicotinamide

For retroviral introduction, a retroviral vector expressing each pluripotency inducer (Oct-3/4, Sox2, KLF4, and c-Myc) was introduced into packaging cells, PLAT-E cells (Kitamura T, et al. (2003) Exp Hematol 31:1007-1014). Culture supernatants of PLAT-E cells were each recovered two days after gene introduction, and filtered through a 0.45 µm cellulose acetate. Polybrene was then added to a final concentration 4 mg/ml, an equal amount of each culture supernatant was mixed, and the mixture was added to Nanog-GFP MEF (Okita K, Ichisaka T, Yamanaka S. (2007) nature 448:313-317) for viral infection. Nanog-GFP MEF is a cell being a mouse embryonic fibroblast (MEF) to which a BAC was introduced, wherein a GFP-IRES-Puro^{r} cassette is inserted into the BAC carrying the mouse Nanog gene. Nanog-GFP MEF expresses GFP in cells having pluripotency but do not express GFP after differentiation.
On Day 4 after retroviral infection, these were seeded on STO feeder cells with suppressed proliferation ability from mitomycin C (SIGMA: M4287) treatment (seeded at 2 x 10³ cells/ml for cells infected with 4F (c-Myc), 3.5 x 10⁴ cells/ml for 3F), and on the next day cultured in mouse ES cell medium (Nishimoto M, et al. (1999) Mol Cell Biol 19:5453-5465). Thereafter the medium was replaced every two days, replaced with puromycin (Puro)-containing mouse ES cell medium (Puro to a final concentration of 1.2 mg/ml) on Day 17 after infection, and cultured for eight more days. Alkaline phosphatase (AP) staining was then performed. Nicotinamide was added simultaneously with retroviral infection to a final concentration 4 mM, and then added at the same concentration for every medium exchange.

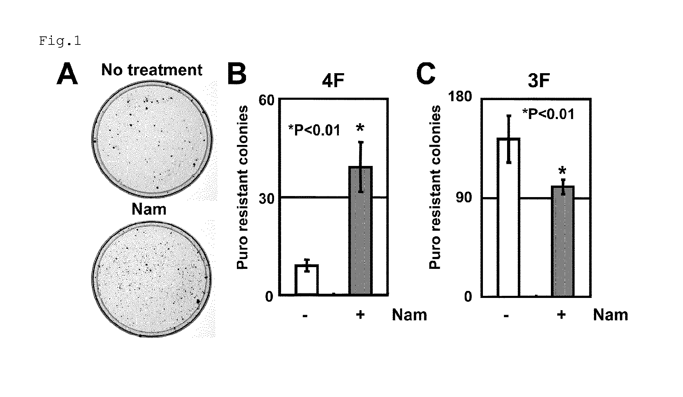

Figure 1A shows the result of Puro-resistant AP-positive colonies when 2 x 10⁴ cells infected with 4F (c-Myc) were seeded in a 10cm² dish, and Figure 1B shows the average number of Puro-resistant AP-positive colonies after 4 x 10³ cells infected with 4F (c-Myc) were seeded in three 3.5 cm² dishes. The error bars show standard deviations. In cells infected with 4F (c-Myc), the number of colonies showing resistance to puromycin significantly increased by administration of nicotinamide.
Figure 1C shows the average number of Puro-resistant AP-positive colonies after 7 x 10⁴ cells infected with 3F were seeded in three 3.5 cm² dishes. The error bars show standard deviations. In cells infected with 3F, increase in induction efficiency by nicotinamide was not seen.
Accordingly, it was confirmed that the induction efficiency of iPS cells increases by nicotinamide, and that introduction of the c-Myc gene is necessary to obtain the effect of nicotinamide.

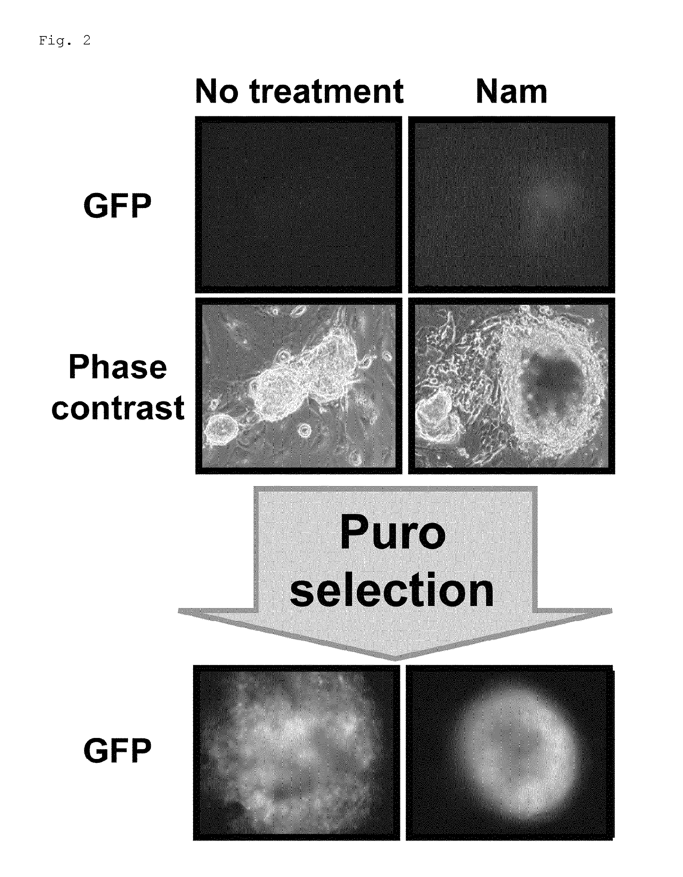

Figure 2 shows the result of observing Nanog-GFP MEF infected with 4F (c-Myc) with a fluorescence microscope. The experimental method is in accordance with Test 1, but the photograph of GFP observation in the upper column shows the colony without drug selection by Puro, and the photograph of GFP observation in the lower column shows the colony after drug selection by Puro. As shown in the upper colume of Figure 2, cells introduced with the pluripotency inducer showed weak color even if cultured in the presence of nicotinamide (Nam), although cells treated with nicotinamide displayed slightly more intense color development compared to nicotinamide-untreated control cells. However, when puromycin was added, a more intense color development was observed in the nicotinamide administration group than the non-administration group. Accordingly, it was found that although the number of colonies significantly increased when nicotinamide was added, and true iPS cells increased along with it, partial iPS cells that are in an incomplete reprogramming state were also included among the increased colony.

### Test 2. Optimization of Nicotinamide Treatment

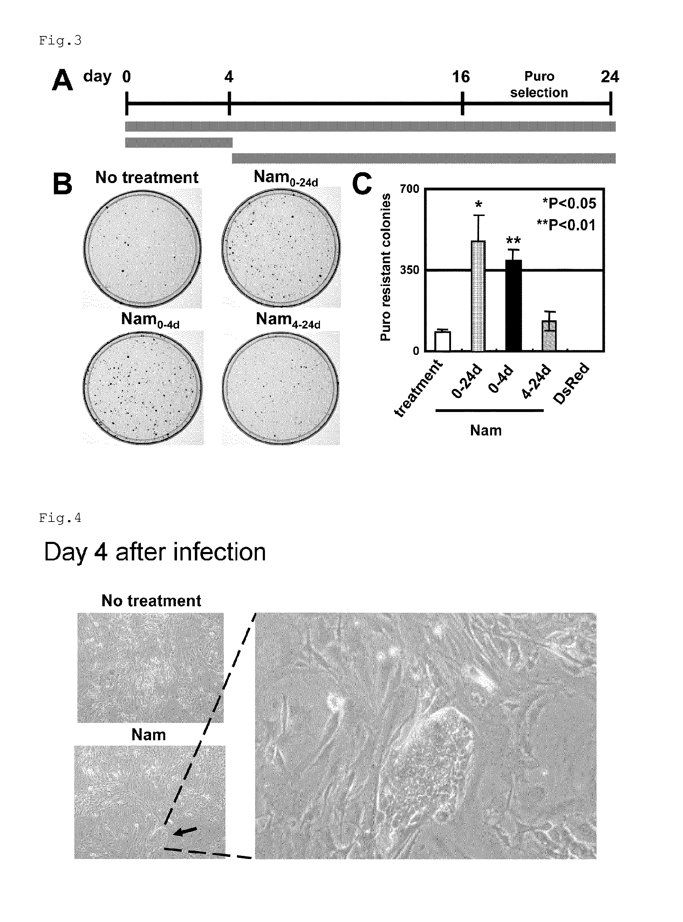

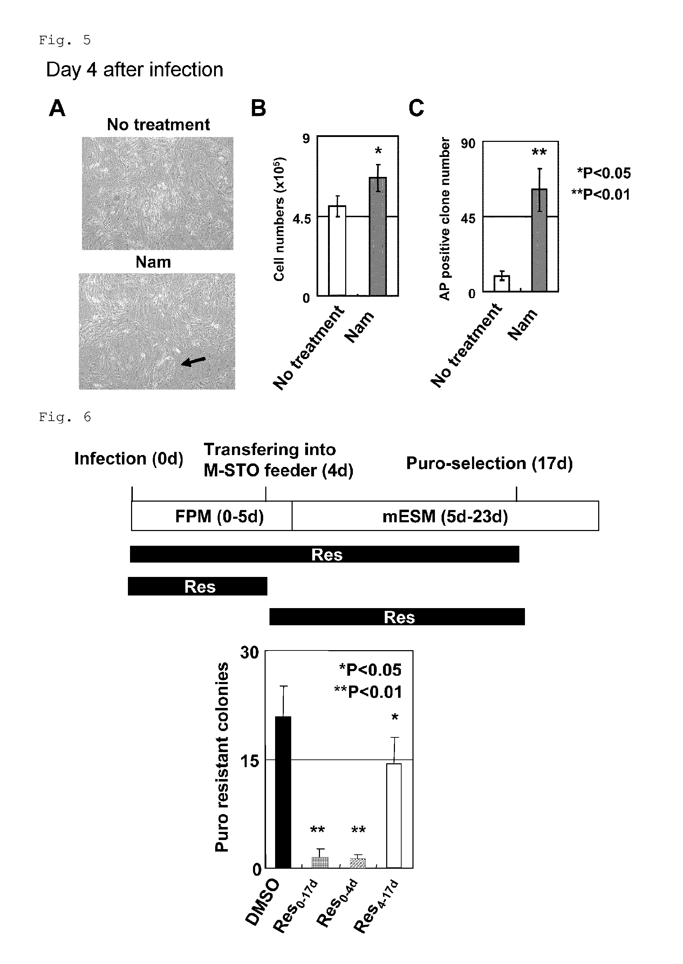

In order to investigate the optimal treatment time of nicotinamide, nicotinamide was added under each conditions of until Day 24 after retroviral infection (Nam 0-24d), until Day 4 after retroviral infection (Nam 0-4d), and from Day 4 to Day 24 after retroviral infection (Nam 4-24d), and iPS cells were induced by 4F (c-Myc). Experimental method other than the nicotine treatment time was in accordance with Test 1.
The result is shown in Figure 3. When nicotinamide was added from Day 0 to Day 24 after introduction of the pluripotency inducer, Puro-resistant colonies had significantly increased. Moreover, compared to addition from Day 0 to Day 24, no great difference was seen in the number of Puro-resistant colonies when the addition was from Day 0 to Day 4, which had similarly significantly increased. On the other hand, when nicotinamide was added on or after Day 4 after introduction of the pluripotency inducer, the effect of increasing the number of Puro-resistant colonies by nicotinamide was barely obtained. From these results, it was revealed that the effect of elevation on the number of Puro-resistant colonies is obtained by nicotinamide treatment at an early stage of iPS cell induction.
Since it was found that only four day's treatment of nicotinamide was sufficient for obtaining its full effect, the experiment was set up to investigate whether the influence of nicotinamide is recognized on Day 4 after retroviral infection by inspecting nicotinamide-treated and -untreated cells at this early point. As a result, iPS cell-like colonies were recognized in the Nam treatment group, but not in the untreated control group. (Figures 4 and 5A).
Next, in order to investigate the influence of nicotinamide on Day 4 after infection, the number of cells was counted and AP staining was performed. As a result, increase in the number of cells was recognized in the nicotinamide treatment group compared to the non-treatment cell group at infection Day 4 (Figure 5B), and a significant increase in the number of AP-positive colonies was recognized (Figure 5C). Further, as a result of investigating the expression of various undifferentiated markers, it became clear that the expression of undifferentiated markers was increased compared to the non-treatment group due to nicotinamide treatment (data not shown). Accordingly, it became clear that iPS cell induction was already promoted on Day 4 after infection by nicotinamide treatment.

### Test 3. Investigation of the Mechanism of iPS Cell Induction Effect by Nicotinamide

Nicotinamide is known to suppress PARP or sirtuin. Consequently, resveratrol which activates sirtuin (SIRT1) was added to the medium instead of nicotinamide to investigate the induction effect of iPS cells. Experimental method was in accordance with Test 2, except that resveratrol (Res) was added to the medium to a final concentration 100 µM.
The result is shown in Figure 6. When resveratrol was added from Day 0 to Day 17 and from Day 0 to Day 4 after introduction of the pluripotency inducer, the number of Puro-resistant colonies significantly decreased compared to the control of adding an equal amount of DMSO, a solvent for resveratrol. On the other hand, when the addition was on Day 4 after introduction of the pluripotency inducer, a relatively large number of Puro-resistant colonies were induced. Accordingly, suppression of sirtuin by nicotinamide underlie its promoting effect on iPS cell induction.

### Test 4. Induction of iPS Cells in the Presence of MEK Inhibitor and GSK-3 Inhibitor (2i Method)

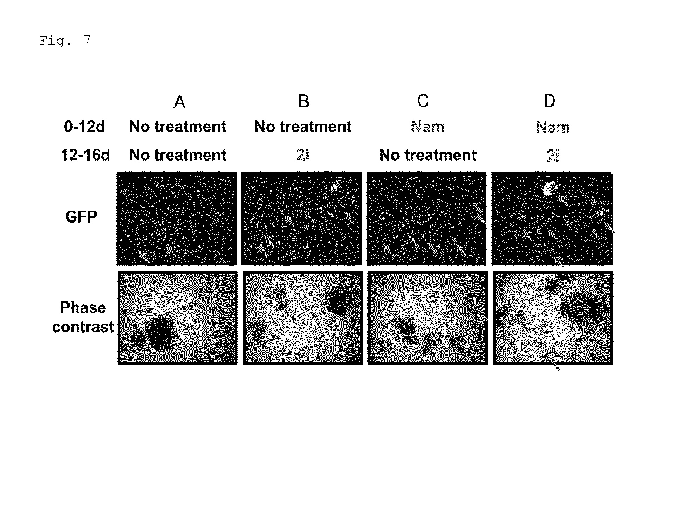

Next, the method of culturing in the presence of an MEK inhibitor and a GSK-3 inhibitor known to promote the reprogramming of partial iPS cells (2i method) was combined with culturing in the presence of nicotinamide.
Nanog-GFP MEF was subjected to forced expression of 4F(c-Myc) retrovirally. These were seeded on feeder cells on Day 4 after retroviral infection, and cultured in mouse ES cell medium from the following day. This was replaced with a serum-free mouse ES cell medium comprising PD0325901 (1 µM) and CHIR99021 (3 µM) (2i medium) from Day 12 after infection. Culturing was then continued for four days, and cells after culturing were observed with a fluorescence microscope. As the control zone, culturing was also performed in a serum-free mouse ES cell medium without PD0325901 and CHIR99021 from 12 days after infection for four days. The result is shown in Figure 7. It was confirmed that true iPS cells are increased by the 2i method (Figures 7A and 7B). In addition, it was confirmed that when the 2i method is performed after treatment with nicotinamide, the number of colonies including partial iPS cells are first increased by nicotinamide, and partial iPS are transformed into true iPS cells by nicotinamide as well as the MEK inhibitor and GSK-3 inhibitor, resulting in a dramatic increase in true iPS cells (Figures 7C and 7D).

### Test 5. Optimization of Conditions for 2i Method

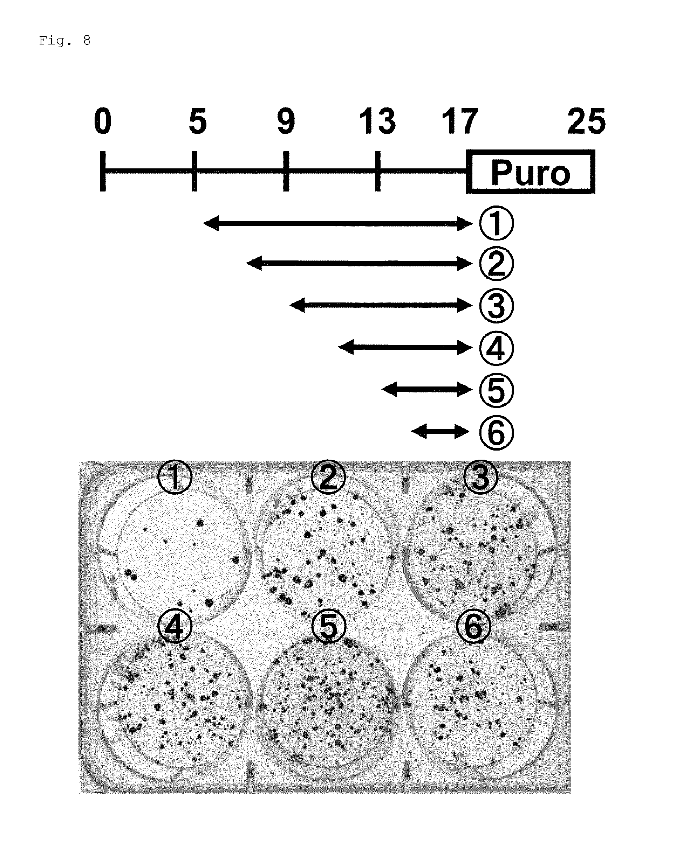

In order to optimize the effect by the 2i method, the timing of adding the MEK inhibitor and GSK-3 inhibitor to the medium was investigated.
Nanog-GFP MEF was subjected to forced expression of 4F(c-Myc) retrovirally. These were seeded on feeder cells on Day 4 after retroviral infection, and cultured in mouse ES cell medium from the following day until replaced with the 2i medium. Culturing in the 2i medium was initiated on (1) Day 5, (2) Day 7, (3) Day 9, (4) Day 11, (5) Day 13, and (6) Day 15 after retroviral infection, and continued until Day 17 after infection. From Day 17 after infection, drug selection by Puro was performed for eight days, and then AP staining was performed. The result is shown in Figure 8. The largest number of Puro-resistant AP-positive colonies was obtained when the 2i method was performed from Day 13 ((5)) to Day 17 (Figure 8).

### Test 6. Induction of iPS Cells by Combination of Nicotinamide and 2i Method

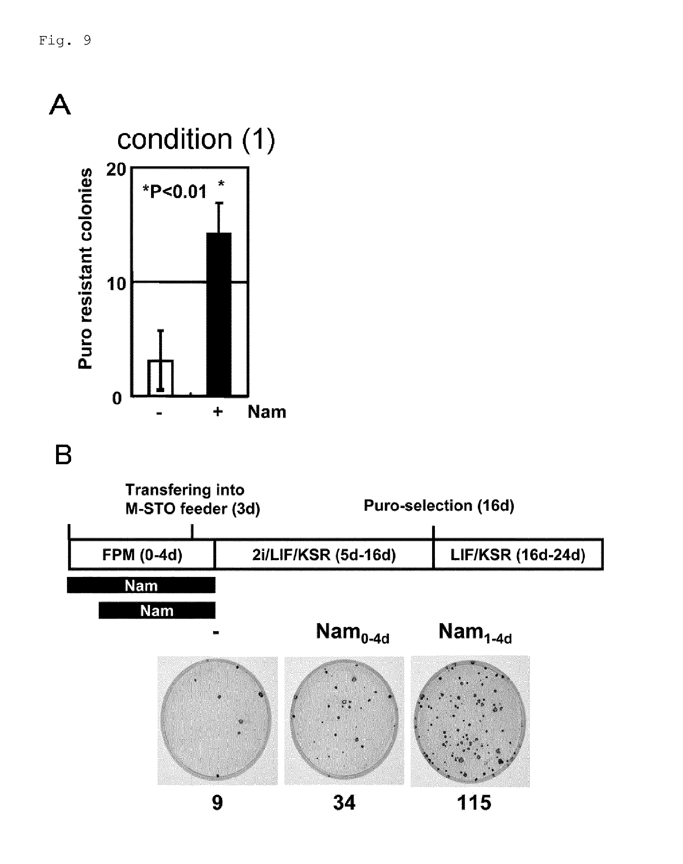

When the 2i method was performed under similar conditions as Test 5 (1), and cultured with the addition of nicotinamide (final concentration of 4 mM) from Day 0 to Day 4 after retroviral infection, the proportion of true iPS cells significantly increased (Figure 9A).
Next, in order to compare when the nicotinamide treatment is performed simultaneously with retroviral infection and when it is performed after one day post infection, investigation was performed in accordance with the conditions of Test 5 (1). As a result, as shown in Figure 9B, it was confirmed that the induction efficiency was higher by three-fold when the addition of nicotinamide was from Day 1 to Day 4 than from Day 0 to Day 4 after retroviral infection.

### Test 7. Induction of Human iPS cells in the Presence of Nicotinamide

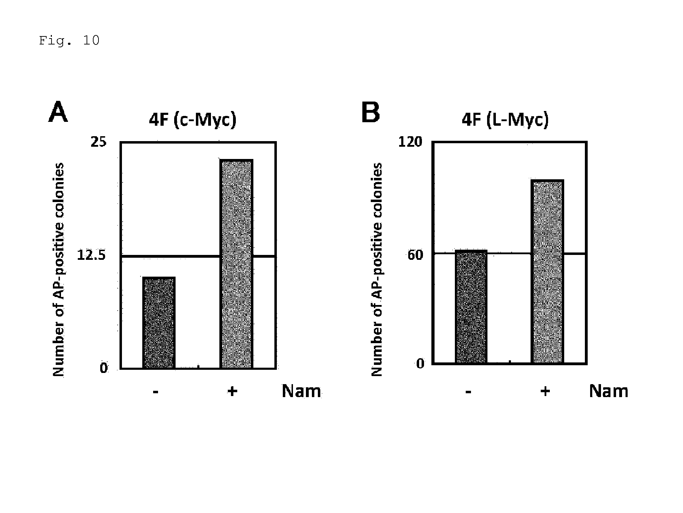

For retroviral introduction, retroviral vectors expressing each pluripotency inducer (Oct-3/4, Sox2, KLF4, and c-Myc) were introduced into packaging cells, PLAT-GP cells (Morita S, et al. (2000) Gene Therapy 7:1063-1066). Culture supernatants of PLAT-GP cells were each recovered two days after gene introduction, and filtered through a 0.45 µm cellulose acetate. Polybrene was then added to a final concentration 4 mg/ml, an equal amount of each culture supernatant was mixed, and the mixture was added to human fibroblasts for viral infection. These were seeded on feeder cells on Day 6 after retroviral infection (4F (c-Myc): 2.5 x 10⁴; 4F (L-Myc): 5.0 x 10⁴), and cultured in human ES cell medium on the following day (Takahashi K, et al (2007) Cell 131:861-872). Thereafter the medium was exchanged every two days, and cultured until thirty days after infection. Alkaline phosphatase (AP) staining was then performed, and the number of AP-positive colonies was counted. Nicotinamide was added simultaneously with retroviral infection to a final concentration 4 mM, and then added at the same concentration for every medium exchange until Day 6 after infection. The control zone was cultured to thirty days after infection without adding nicotinamide. The result is shown in Figure 10.
As shown in Figure 10, the number of AP-positive colonies increased in the zone with the addition of nicotinamide compared to the zone without the addition of nicotinamide.
As described above, similar to mouse fibroblasts, the efficiency of AP-positive colony induction could also be improved by culturing in the presence of a sirtuin inhibitor and/or a PARP inhibitor in human fibroblasts (Figure 10A). Moreover, when the L-Myc gene as the Myc family gene was employed as the pluripotency inducer, the induction efficiency of AP-positive colonies could also be improved by culturing in the presence of a sirtuin inhibitor and/or a PARP inhibitor (Figure 10B).

## Claims

1. A method for producing an artificial pluripotent stem cell (iPS cell) comprising the steps of:
introducing a pluripotency inducer comprising at least a Myc family gene or a Myc family protein into a somatic cell; and
culturing said somatic cell in the presence of a sirtuin inhibitor and/or a poly-ADP ribose polymerase (PARP) inhibitor.

2. The method according to claim 1, wherein the step of culturing said somatic cell in the presence of said sirtuin inhibitor and/or PARP inhibitor is, counting the day of performing the step of introducing said pluripotency inducer into the somatic cell as Day 0, terminated before Day 10.

3. The method according to claim 1 or 2, wherein the step of culturing said somatic cell in the presence of said sirtuin inhibitor and/or PARP inhibitor is, counting the day of performing the step of introducing said pluripotency inducer into the somatic cell as Day 0, initiated on or after Day 1.

4. The method according to any one of claims 1 to 3, wherein said sirtuin inhibitor and/or PARP inhibitor is nicotinamide.

5. The method according to claim 4, wherein the step of culturing said somatic cell in the presence of said sirtuin inhibitor and/or PARP inhibitor comprises adding said nicotinamide at a concentration of 1 mM to 10 mM to a medium for said somatic cell.

6. The method according to any one of claims 1 to 5, which comprises, after the step of culturing said somatic cell in the presence of said sirtuin inhibitor and/or PARP inhibitor,
a step of further culturing said somatic cell in the presence of a GSK-3 inhibitor and a MEK inhibitor.

7. The method according to claim 6, wherein the step of further culturing said somatic cell in the presence of said GSK-3 inhibitor and MEK inhibitor is, counting the day of performing the step of introducing said pluripotency inducer into the somatic cell as Day 0, initiated on or after Day 5.

8. The method according to claims 1 to 7, wherein said Myc family gene or Myc family protein is a c-Myc gene or L-Myc gene, or a c-Myc protein or L-Myc protein.

9. The method according to any one of claims 1 to 8, wherein said pluripotency inducer further comprises one or more genes selected from the group consisting of Oct3/4 gene, Klf4 gene, Sox2 gene, Nanog gene and LIN28 gene.

10. A method for producing an artificial pluripotent stem cell (iPS cell), comprising the steps of:
introducing a pluripotency inducer into a somatic cell; and
further culturing said somatic cell in the presence of a GSK-3 inhibitor and a MEK inhibitor;
wherein the step of further culturing said somatic cell in the presence of said GSK-3 inhibitor and MEK inhibitor is, counting the day of performing the step of introducing said pluripotency inducer into the somatic cell as Day 0, initiated on or after Day 5.

11. The method according to claim 10, wherein said pluripotency inducer is one or more genes selected from the group consisting of Oct3/4 gene, Klf4 gene, Sox2 gene, c-Myc gene, L-Myc gene, Nanog gene, and Lin28 gene.

12. The method according to claims 1 to 11, wherein said artificial pluripotent stem cell (iPS cell) is a mouse or human-derived cell.

13. An artificial pluripotent stem cell (iPS cell) produced by the method according to any one of claims 1 to 12.

14. A culture kit for an artificial pluripotent stem cell (iPS cell), comprising:
a sirtuin inhibitor or poly-ADP ribose polymerase (PARP) inhibitor;
a GSK-3 inhibitor; and
an MEK inhibitor.
